# EUROPÄISCHE PATENTANMELDUNG

(11) **EP 0 836 855 A1**
(43) Veröffentlichungstag der Anmeldung: **22.04.1998**
(21) Anmeldenummer: 96116599.0
(22) Anmeldetag: 16.10.1996
(51) Int. Cl.: A61L 9/01

(54) **Sauna bzw. Dampfbade-Essenz**

(71) Anmelder: SCHUPP GmbH & Co., 72238 Freudenstadt (DE)
(72) Erfinder: Schupp, Eduard, D-72250 Freudenstadt (DE)
(74) Vertreter: Säger, Manfred, Dipl.-Ing.

(57) **Zusammenfassung**

Eine Sauna-bzw. Dampfbade-Essenz enthält:
3 bis 60 Gew.-% etherische Öle bzw. Parfümöle;
0 bis 20 Gew.-% Wasser;
0 bis 10 Gew.-% Emulgator und
Rest Alkylenglykole.

## Beschreibung

Die vorliegende Erfindung betrifft eine Sauna- bzw. Dampfbade-Essenz gemäß dem Hauptanspruch.

Die Herstellung von Sauna-Zusatz-Essenzen bzw. Sauna-Konzentraten oder Dampfbade-Essenzen ist problematisch, da die hierfür eingesetzten etherischen Öle bzw. Parfümöle brennbar sind bzw. verpuffen können. Sie gehören im allgemeinen der Brandklasse II an. Durch solche Zusätze sind in der Vergangenheit schon vielfach schwere Sauna-Brände, zum Teil mit schwersten Körperverletzungen bzw. Brandverletzungen entstanden.

Die Brandgefahr solcher Essenzen wird noch dadurch erhöht, daß häufig neben dem eigentlichen etherischen Öl z.B. der indischen Melisse (Citronellöl), reines Limonen oder doppelt rektifiziertes Terpentinöl zur Verdünnung genommen wird, wobei gerade diese beiden letztgenannten Lösungsmittel die Brandgefahr weiter erhöhen. Die herkömmlichen Sauna-Esszenzen enthalten in der Regel 10 bis 50 % etherische Öle bzw. Parfümöle, wobei zum Verdünnen häufig Alkohol oder ähnliche Lösungsmittel verwendet werden, welche die Brandgefahr der etherischen Öle noch erhöhen.

Aufgabe der vorliegenden Erfindung ist es, eine Sauna- bzw. Dampfbade-Essenz zu schaffen, die trotz des Einsatzes der brennbaren etherischen Öle bei den in Saunen eingesetzten Verdünnungen nicht brennbar ist, so daß die Gefahr von Verpuffungen der brennbaren Anteile vermieden wird.

Es wurden bereits mit Wasser verdünnte Essenzen in den Handel gebracht, die zur Vermittlung der Wasserlöslichkeit einen hohen Anteil an Emulgatoren haben. Diese Essenzen haben jedoch den Nachteil, daß die Emulgatoren auf den heißen Steinen oder der glühenden Herdplatte verbrennen und dabei einen brenzlich unangenehmen Geruch hervorrufen, der vom Sauna-Benutzer als unangenehm empfunden wird und zu Hustenreizen, ja sogar bis zu Ohnmachten führen kann. Gesundheitsrisiken durch unbekannte Crack-Produkte sind ebenso möglich.

Aus diesem Grunde soll der Anteil an Emulgatoren bei der erfindungsgemäßen Sauna- bzw. Dampfbade-Essenz möglichst niedrig gehalten werden. Er soll auf jeden Fall unter 10 % betragen, vorzugsweise aber unter 5 %, insbesondere unter 2 % und ganz besonders bevorzugt innerhalb einer Mengenbegrenzung von 0,0 bis 1 % liegen.

Es hat sich gezeigt, daß Essenzen, die bei den in der Sauna üblichen Verdünnungen nicht mehr brennbar sind bzw. nicht verpuffen, hergestellt werden können, wenn Alkylenglykole mit Wasser mit einem eventuell geringen Zusatz eines Emulgators mit dem etherischen Öl bzw. Parfümöl vermischt werden. Solche Essenzen bilden auch beim Verdünnen mit Wasser, wobei dessen Anteil mindestens 75 % betragen soll, also unter den üblichen Sauna-Bedingungen, stabile Emulsionen, bei denen das etherische Öl nicht aufrahmt und daher eine Verpuffungsgefahr ausgeschlossen ist. Durch den geringen bzw. auf Null reduzierten Emulgatoranteil setzen sie auch bei der Verdampfung auf den Sauna-Steinen keine gesundheitsgefährdenden Stoffe frei, so daß ihre Anwendung auch bei empfindlichen Personen möglich ist.

Als etherische Öle bzw. Parfümöle können alle für diesen Zweck bisher verwendeten Stoffe unabhängig von ihrer Brennbarkeit eingesetzt werden. Infolge der universellen Einsetzbarkeit aller dieser Öle eröffnet sich für den Anwender der Sauna-Essenzen eine unerschöpfliche Vielfalt an möglichen Geruchskombinationen.

Der Wasseranteil der Essenz kann bis 20 Gew.-% betragen, vorzugsweise kann er auf bis zu 10 % beschränkt werden.

Als Lösungsmittel der Sauna-Essenz dienen Alkylenglykole, wie beispielsweise Ethylen- oder Propylenglykole, z.B. Propylenglykol, Dipropylenglykol, Triethylenglykol oder andere niedere Alkylenglykole, die bei Normalbedingungen flüssig sind.

Als Emulgatoren werden vorzugsweise ethoxylierte Fettsäuren bzw. Fettalkohole und/oder Fette und Öle bzw. deren Derivate eingesetzt, die unter den in der Sauna herrschenden heißen Bedingungen keine gesundheitsgefährdenden Stoffe bei ihrer Zersetzung abgeben. Es handelt sich vorzugsweise um zur Verbindungsklasse der Polyether gehörende Polyalkylenglykole, die als Lösungsvermittler bzw. Emulgatoren im Handel sind. Solche Stoffe sind beispielsweise Polyoxyethylen-(20)-Sorbitanmonooleat (Polysorbat 80), Polyoxyethylen-(20)-Sorbitanmonolaurat (Polysorbat 20) sowie die entsprechenden Palmitate bzw. Stearate.

Die erfindungsgemäßen Sauna-Esszenzen werden zum Einsatz in der Sauna mit Wasser verdünnt, wobei eine Verdünnung 75 % und mehr möglich ist. In diesen Verdünnungen bildet sich mit der Sauna-Essenz eine stabile Emulsion aus, in der sich die brennbaren Bestandteile nicht abscheiden.

Die Erfindung wird nachfolgend anhand der Beispiele näher erläutert.

### BEISPIEL 1

Die folgenden Bestandteile werden zu einer Sauna-Essenz verarbeitet:
- 30 Gew.-%: naturreines Eukalyptusöl
- 7 Gew.-%: Wasser
- 1 Gew.-%: Polysorbat 80 (Polyoxyethylen-20-Sorbitanmonooleat)
- 62 Gew.-%: Dipropylenglykol.

Die Mischung bildet eine stabile Emulsion, die auch beim Verdünnen mit 75 % Wasser oder mehr stabil bleibt und nicht aufrahmt.

## Patentansprüche

1. Sauna-bzw. Dampfbade-Essenz, enthaltend
3 bis 60 Gew.-% etherische Öle bzw. Parfümöle;
0 bis 20 Gew.-% Wasser;
0 bis 10 Gew.-% Emulgator; und
Rest Alkylenglykole.

2. Essenz nach Anspruch 1, dadurch gekennzeichnet, daß die Menge des Emulgators bis 5 Gew.-% beträgt.

3. Essenz nach Anspruch 1, dadurch gekennzeichnet, daß die Menge des Emulgators 0,5 bis 2 Gew.-% beträgt.

4. Essenz nach Anspruch 1, dadurch gekennzeichnet, daß die Menge des Emulgators 0,5 bis 1 Gew.-% beträgt.

5. Essenz nach einem der vorherigen Ansprüche, dadurch gekennzeichnet, daß der Emulgator ausgewählt ist aus der Gruppe ethoxylierter Fettsäuren bzw. Fettalkohole und/oder hydrierter Fette und Öle bzw. deren Derivate.

6. Essenz nach einem der vorherigen Ansprüche, dadurch gekennzeichnet, daß die Menge des Alkylenglykols bis 97 %, vorzugsweise bis 93,5 Gew.-% beträgt.

7. Essenz nach einem der vorherigen Ansprüche, dadurch gekennzeichnet, daß die eingesetzte Wassermenge innerhalb der Löslichkeitsgrenze des eingesetzten etherischen Öle bzw. Parfümöls liegt.
